# EUROPEAN PATENT APPLICATION

(11) **EP 3 300 660 A1**
(43) Date of publication of application: **04.04.2018**
(21) Application number: 17193678.4
(22) Date of filing: 28.09.2017
(51) Int. Cl.: A61B 5/00, A61B 18/14, A61B 5/0402

(54) **BASKET CATHETER CONFORMING TO ORGAN USING STRAIN-RELIEF ELEMENTS**

(30) Priority: 29.09.2016 US 201615279570
(71) Applicant: Biosense Webster (Israel), Ltd., Yokneam, 2066717 (IL)
(72) Inventor: AUERBACH, Shmuel, 2066717 Yokneam (IL); RAVUNA, Eliyahu, 2066717 Yokneam (IL); BOTZER, Lior, 2066717 Yokneam (IL)
(74) Representative: Carpmaels & Ransford LLP

(57) **Abstract**

A catheter includes a shaft for insertion into an organ of a patient, and an extendable distal-end assembly. The distal-end assembly is coupled to the shaft and includes multiple splines. At least one spline includes one or more electrodes and at least one strain-relief element that is configured, when the distal-end assembly is extended in the organ, to allow the spline to conform to a surface of the organ so as to make contact between the electrodes and the surface.

## Description

### FIELD OF THE INVENTION

The present invention relates generally to intrabody medical probes, and particularly to catheters having mechanical strain-relief elements.

### BACKGROUND OF THE INVENTION

Basket catheters may be used in various medical applications, such as cardiology. Several types of basket catheters, having multiple splines, are designed to enable sensing and treating of arrhythmia.

For example, U.S. Patent Application Publication 2014/0303469, whose disclosure is incorporated herein by reference, describes a method for sensing multiple local electric voltages from endocardial surface of a heart. The method includes: providing a system for sensing multiple local electric voltages from endocardial surface of a heart, including: a first elongate tubular member having a lumen, a proximal end and a distal end; a basket assembly including: a plurality of flexible splines for guiding a plurality of exposed electrodes, the splines having proximal portions, distal portions and medial portions therein between, wherein the electrodes are substantially flat electrodes and are substantially unidirectionally oriented towards a direction outside of the basket.

U.S. Patent 8,337,492, whose disclosure is incorporated herein by reference, describes devices, systems and methods for the mapping of electrical signals and the ablation of tissue. Embodiments include an ablation catheter that has an array of ablation elements attached to a deployable carrier assembly. The carrier assembly can be transformed from a compact, linear configuration to a helical configuration, such as to map and ablate pulmonary vein ostia.

U.S. Patent 8,825,130, whose disclosure is incorporated herein by reference, describes an electrode support structure assembly that comprises an electrode support structure including a plurality of splines. Each of the plurality of splines can have a proximal end portion and a distal end portion. The assembly further comprises a first element defining an axis and comprising an outer surface. The outer surface comprises a plurality of slots configured to receive the distal end portion of each of the plurality of splines. The first element is configured such that the distal end portion of each of the plurality of splines may move with respect to each slot.

### SUMMARY OF THE INVENTION

An embodiment of the present invention that is described herein provides a catheter including a shaft for insertion into an organ of a patient, and an extendable distal-end assembly. The distal-end assembly is coupled to the shaft and includes multiple splines. At least one spline includes one or more electrodes and at least one strain-relief element that is configured, when the distal-end assembly is extended in the organ, to allow the spline to conform to a surface of the organ so as to make contact between the electrodes and the surface.

In some embodiments, the strain-relief element includes nitinol. In other embodiments, in response to being under a mechanical stress, the strain-relief element is configured to allow the spline to conform to the surface. In yet other embodiments, the strain-relief element has a shape selected from a list consisting of S-shape, U-Shape, Omega-shape, V-Shape, and Double V-shape.

In an embodiment, the strain-relief element is positioned in a proximal-end of the at least one spline. In another embodiment, the strain-relief element is positioned in a distal-end of the at least one spline. In yet another embodiment, the at least one spline includes at least one strain-relief element positioned at a proximal-end of the at least one spline, and at least one strain-relief element positioned at a distal-end of the at least one spline.

In some embodiments, a volume of the extended distal-end assembly is larger than a volume of the organ, and the strain-relief element is configured to partially collapse so as to allow the splines to conform to the surface. In other embodiments, a volume of the extended distal-end assembly is smaller than a volume of the organ, and the strain-relief element is configured to extend so as to allow the splines to conform to the surface.

There is additionally provided, in accordance with an embodiment of the present invention, a method for producing a catheter. The method includes providing a shaft for insertion into an organ of a patient. An extendable distal-end assembly, which includes multiple splines of which at least one spline includes one or more electrodes and at least one strain-relief element is coupled to the shaft.

There is additionally provided, in accordance with an embodiment of the present invention, a method for applying a medical procedure. The method includes inserting a catheter shaft into an organ of a patient. A distal-end assembly, which is coupled to the shaft, is extended inside the organ. The distal-end assembly includes multiple splines, of which at least one spline includes one or more electrodes and at least one strain-relief element, so as to allow the spline to conform to a surface of the organ and to make contact between the electrodes and the surface. The medical procedure is applied using the electrodes.

The present invention will be more fully understood from the following detailed description of the embodiments thereof, taken together with the drawings in which:

### BRIEF DESCRIPTION OF THE DRAWINGS

Fig. 1 is a schematic, pictorial illustration of a catheter tracking system, in accordance with an embodiment of the present invention;
Figs. 2 and 3 are schematic, pictorial illustrations of distal-end assemblies in an extended position, in accordance with embodiments of the present invention; and
Figs. 4A-4D are schematic, pictorial illustrations of alternative strain-relief element configurations, in accordance with other embodiments of the present invention.

### DETAILED DESCRIPTION OF EMBODIMENTS

### OVERVIEW

Embodiments of the present invention that are described hereinbelow provide improved distal-end assemblies for medical catheters. In some embodiments, a distal-end assembly of a catheter comprises multiple splines having various kinds of electrodes. The catheter is typically inserted to a human body in a collapsed position (e.g., using a sheath) and extended upon reaching a cavity of an organ in question. In the extended position, it is important that the splines will be able to fully conform to the desired shape of the cavity so that the catheter electrodes will make contact with the organ surface.

In some embodiments, at least one of the splines comprises one or more strain-relief elements. The strain-relief elements are configured, when the distal-end assembly is extended in the cavity, to reduce mechanical strain in the splines, and allow the splines (and thus the electrodes) to better conform to the surface of the cavity.

In an embodiment, the strain-relief element may be fabricated from a super-elastic material such as nitinol, wherein in the presence of mechanical stress, the strain-relief element enables the spline to conform to the surface of the cavity. In an embodiment, the strain-relief element may be positioned at any suitably selected point along the spline, such as at the proximal end, or at the distal-end assembly. In another embodiment, each of the splines may comprise one or more strain-relief elements, wherein different splines may comprise a different number of spline-elements. In yet another embodiment, at least one of the splines may not comprise any strain-relief elements.

The disclosed techniques are particularly effective in multi-spline catheters, which typically comprise a large number of electrodes and sensors for treating various types of cavities. A lack of sufficient mechanical contact between the entire lengths of the splines and the surface of the cavity would severely degrade the quality of the mapping and/or treatment. The disclosed techniques overcome this limitation and enable the catheter to fit the organ shape using any desired number of splines, electrodes and sensors, thereby obtaining high quality treatment and shortening the procedure cycle time. The disclosed techniques can be used with various multi-spline structures, such as expandable basket catheters or any other suitable configuration in a wide range of medical applications.

### SYSTEM DESCRIPTION

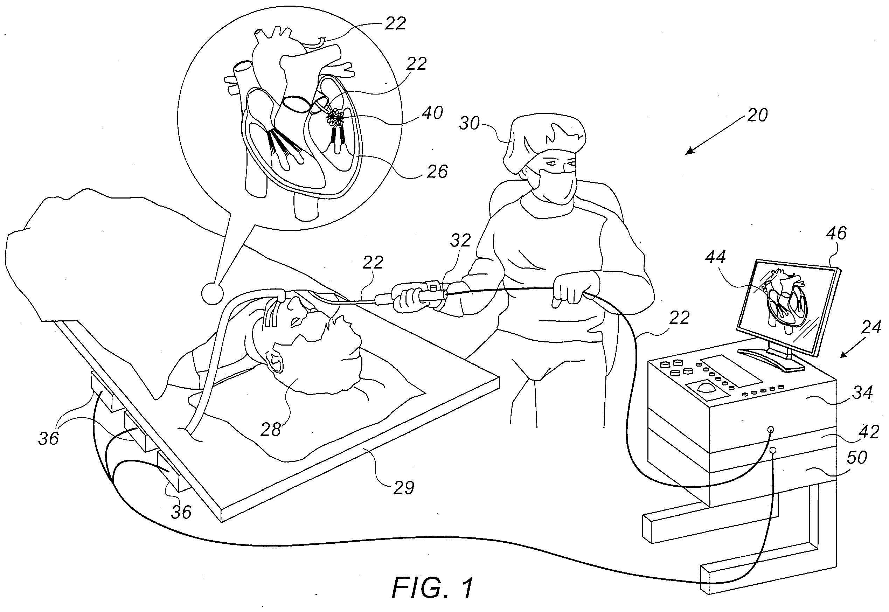

Fig. 1 is a schematic, pictorial illustration of a catheter tracking system 20, in accordance with an embodiment of the present invention. System 20 comprises a probe 22, in the present example a cardiac catheter, and a control console 24. In the embodiment described herein, catheter 22 may be used for any suitable therapeutic and/or diagnostic purposes, such as ablation of tissue in a heart 26 and the mapping of electro-cardiac signals for the diagnosis of cardiac dysfunctions, such as cardiac arrhythmias, for example.

Console 24 comprises a processor 42, typically a general-purpose computer, with suitable front end and interface circuits for receiving signals from catheter 22 and for controlling the other components of system 20 described herein. Processor 42 may be programmed in software to carry out the functions that are used by the system, and the processor stores data for the software in a memory 50. The software may be downloaded to console 24 in electronic form, over a network, for example, or it may be provided on non-transitory tangible media, such as optical, magnetic or electronic memory media. Alternatively, some or all of the functions of processor 42 may be carried out by dedicated or programmable digital hardware components.

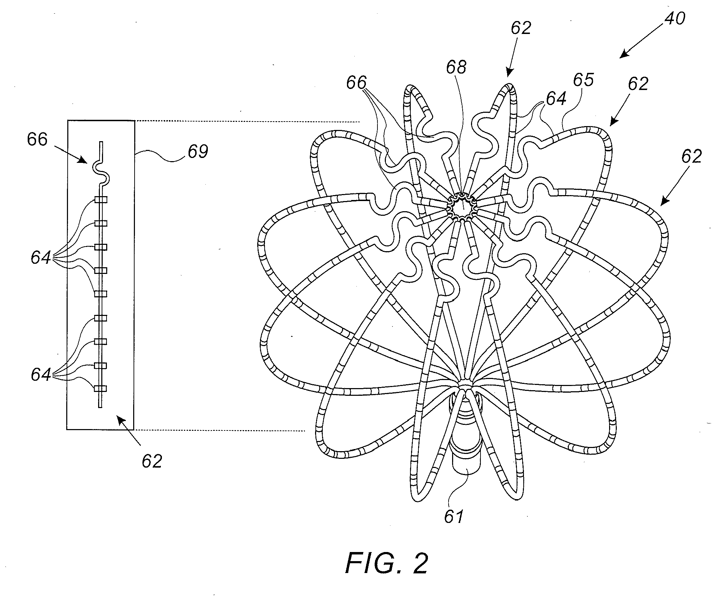

An operator 30 (such as an interventional cardiologist) inserts catheter 22 through the vascular system of a patient 28 lying on a table 29. Catheter 22 comprises an insertion tube, and a distal-end assembly 40 that comprises multiple splines (shown in Fig. 2) . Operator 30 moves assembly 40 of catheter 22 in the vicinity of the target region in heart 26 by manipulating catheter 22 with a manipulator 32 near the proximal end of the catheter as shown in the inset of Fig. 1. The proximal end of catheter 22 is connected to interface circuitry in processor 42.

The position of the distal-end assembly in the heart cavity is typically measured by magnetic position sensing in catheter tracking system 20. In this case, console 24 comprises a driver circuit 34, which drives magnetic field generators 36 placed at known positions external to patient 28 lying on table 29, e.g., below the patient's torso.

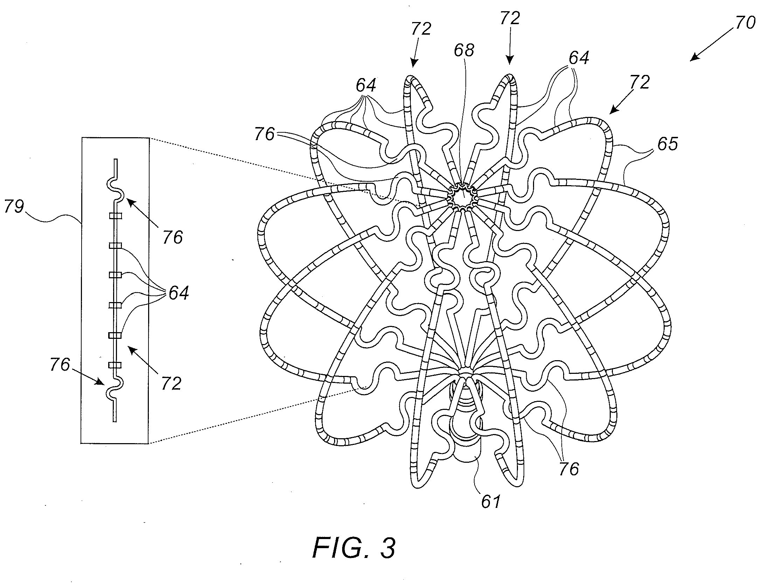

Distal-end assembly 40 typically comprises multiple splines, each comprising one or more magnetic field sensors and/or one or more mapping electrodes (shown in Figs. 2 and 3 below). When the distal-end assembly is brought into contact with the inner heart surface, the mapping electrodes generate potential gradient signals in response to the sensed electrical potentials and the position sensors generate position signals in response to the sensed external magnetic fields, thereby enabling processor 42 to map the electrical potentials as a function of position within the heart cavity.

The multiple magnetic position sensors and mapping electrodes in assembly 40 are connected to interface circuitry in processor 42 at the catheter proximal end. Operator 30 can view the position of assembly 40 on an image 44 of heart 26 on a user display 46.

This method of position sensing is implemented, for example, in the CARTO™ system, produced by Biosense Webster Inc. (Diamond Bar, Calif.) and is described in detail in U.S. Patents 5,391,199, 6,690,963, 6,484,118, 6,239,724, 6,618,612, 6,332,089 and 7,729,742, in PCT Patent Publication WO 96/05768, and in U.S. Patent Application Publication 2004/0068178 A1, whose disclosures are all incorporated herein by reference.

Fig. 2 is a schematic, pictorial illustration of distal-end assembly 40 in extended position, in accordance with an embodiment of the present invention. Assembly 40 comprises one or more splines 62. Each spline 62 comprises a flexible arm that may be fabricated from nitinol, which is a bio-compatible, super-elastic alloy of nickel and titanium, or from any other suitable material that allows bending the splines when assembly 40 is extended. During insertion of the catheter, splines 62 are grouped together in a collapsed position and held in a sheath, or any other suitable device. After insertion into the cavity of interest, the splines are set to an extended position as shown in Fig. 2.

Each spline 62 typically comprises one or more embedded electrodes 64, such as mapping electrodes, position sensors, tissue ablation electrodes, or any other suitable type of electrodes. Spline 62 further comprises a polymer coating 65, which is made of PVAC or any other suitable material, and is wrapped around the arm excluding electrodes 64. At the extended position, one or more of splines 62 are in contact with the inner heart surface in order to collect signals from the heart tissue using the electrodes.

Assembly 40 further comprises a distal fitting 68 located at a distal-end of assembly 40 and a shaft 61, which enables transition of assembly 40 between the collapsed and extended positions. Distal fitting 68 and shaft 61 may be fabricated from nitinol and configured to couple the ends of splines 62. In an extended position, shaft 61 is brought in proximity with distal fitting 68, thereby bending splines 62 as shown in Fig. 2.

In an embodiment, the length of splines 62 is determined by the volume of the cavity in question. For example, when treating a left atrial of a human heart, the diameter of each assembly 40 may be between 35 mm and 80 mm. Typically, the shape of a human cavity (e.g., the left atrium) is not symmetrically round, and therefore at least some of electrodes 64 may not touch the inner wall of the left atrium.

In some embodiments, one or more of splines 62 may comprise at least one strain-relief element 66, which is configured to allow the spline to conform to the surface of the left atrium so as to make contact between electrodes 64 and the surface of the left atrium. In the example of Fig. 2, each spline comprises a single strain-relief element 66, which is positioned close to distal fitting 68.

An inset 69 shows a schematic sectional view of a single spline 62 in a collapsed position. In some embodiments, the total length of spline 62 is 150 mm, or any other suitable length, and multiple electrodes 64 are distributed along the spline. Strain-relief element 66 is positioned, in this example, 10 mm from distal fitting 68 (shown in Fig. 2). In the example of inset 69, element 66 is partially extended, having an S-shape, and adding 20 mm to the total length of spline 62. The width of element 66 in this example is 7 mm, but when fully extended, the width is substantially similar to the width of spline 62 and the length increases accordingly.

In an embodiment, when fully collapsed, element 66 may comprise about 10 percent of the total length of spline 62, although any other suitable ratio may be applied to allow conformity between the splines and the surface of the cavity in question. In other embodiments, some of splines 62 may not comprise any strain-relief elements such as element 66. For example, element 66 may be fitted only in every second spline 62, or covering only one side (e.g., third or half) of assembly 40.

In some embodiments, element 66 may comprise a single s-shape as shown in Fig. 2. In alternative embodiments, element 66 may comprise less than a full s-shape, or a cascade of more than a full s-shape, for example, 0.75, 1.5, 2, or 3 cycles of the s-shape.

Fig. 3 is a schematic, pictorial illustration of a basket catheter assembly 70 in extended position, in accordance with an embodiment of the present invention. Assembly 70 may replace, for example, assembly 40 of Fig. 1 above. Assembly 70 comprises distal fitting 68 and shaft 61, as in assembly 40 depicted in Fig. 2 above. Splines 72 comprise electrodes 64 and coating 65 as also depicted in Fig. 2 above. In the present embodiment, however, one or more of splines 72 comprise two or more strain-relief elements 76. Each element 76 is substantially similar to element 66 depicted in Fig. 2 above.

Reference is now made to an inset 79, which is a schematic, sectional view of a single spline 72 in a collapsed position. In the present non-limiting example, spline 72 comprises a first element 76, which is positioned about 10 mm from the upper end (e.g., from distal fitting 68 shown in Fig. 3) of spline 72, as also depicted for element 66 in inset 69 above. Spline 72 further comprises a second, substantially similar, element 76, which is positioned 13 mm from the lower end (e.g., from shaft 61 shown in Fig. 3). In this arrangement, spline 72 may have better conformity to the surface of the cavity in question (e.g., the left atrium).

In other embodiments, spline 72 may comprise a single element 76, which may be located at any suitable point along the spline so as to allow the required conformity between spline 72 and the surface of the respective tissue. In yet other embodiments, each spline 72 may comprise any suitable number (e.g., greater than two) of elements 76, each located in a suitably selected position along spline 72.

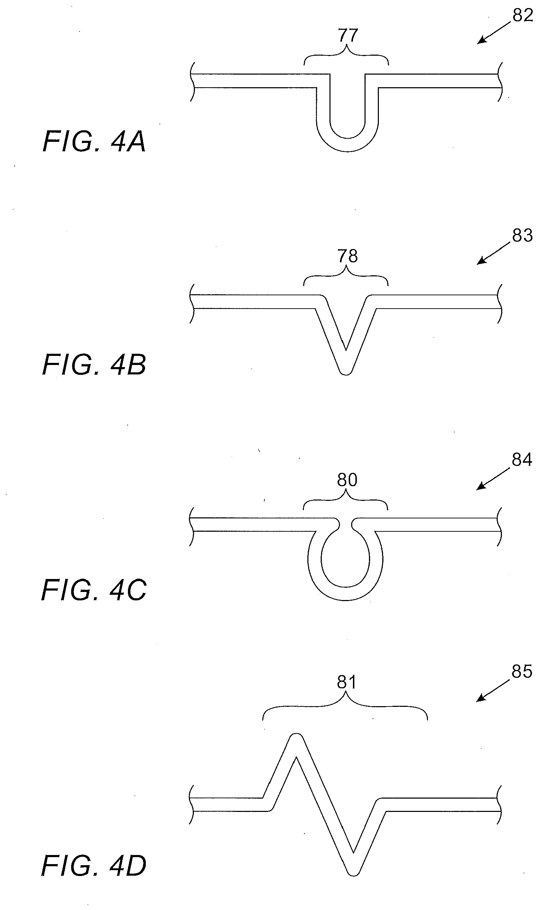

Figs. 4A, 4B, 4C and 4D are schematic, pictorial illustrations of several respective strain-relief element configurations 77, 78, 80 and 81, in accordance with alternative embodiments of the present invention. Splines 82, 83, 84, and 85 comprise strain-relief elements 77 (U-shaped), 78 (V-shaped), 80 (Q-shaped) and 81 (lightning or double-V shaped), respectively. Each strain-relief element among elements 77, 78, 80 and 81 may replace, for example, elements 66 or 76 shown in respective Figs. 2 and 3, above. Elements 77, 78, 80 and 81 provide alternative shape configurations to the previously described S-shaped strain-relief element.

In some embodiments, the alternative configurations may comprise several different types of shapes, such as U-Shaped, Omega-shaped, V-Shaped, and Double V-shaped elements, but may comprise any other suitable shape configuration. Furthermore, each spline among splines 82, 83, 84, and 85, may comprise a series duplication of cycles of the corresponding strain-relief element, as described for element 66 in Fig. 1 above.

In the present context, the term "strain-relief element" refers to any mechanical element that is more laterally-flexible than the spline in which it is fitted, and therefore bends more easily than the spline when subjected to mechanical stress.

Although the embodiments described herein mainly address basket catheters, the methods and systems described herein can also be used in any other suitable medical probe having splines that need to make mechanical contact with tissue.

It will be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

### Aspects of the invention

1. A method for applying a medical procedure, comprising:
   inserting a catheter shaft into an organ of a patient;
   extending inside the organ a distal-end assembly, which is coupled to the shaft and comprises multiple splines, wherein at least one spline comprises one or more electrodes and at least one strain-relief element, so as to allow the spline to conform to a surface of the organ and to make contact between the electrodes and the surface; and
      applying the medical procedure using the electrodes.
2. The method according to aspect 1, wherein extending the distal-end assembly comprises applying the at least one spline to the surface, wherein the at least one strain-relief element is positioned in a proximal-end of the at least one spline.
3. The method according to aspect 1, wherein extending the distal-end assembly comprises applying the at least one spline to the surface, wherein the at least one strain-relief element is positioned in a distal-end of the at least one spline.
4. The method according to aspect 1, wherein extending the distal-end assembly comprises applying the at least one spline to the surface, wherein:
   at least one strain-relief element is positioned at a proximal-end of the at least one spline; and
   at least one strain-relief element is positioned at a distal-end of the at least one spline.

## Claims

1. A catheter, comprising:
a shaft for insertion into an organ of a patient; and
an extendable distal-end assembly, which is coupled to the shaft and comprises multiple splines, wherein at least one spline comprises one or more electrodes and at least one strain-relief element that is configured, when the distal-end assembly is extended in the organ, to allow the spline to conform to a surface of the organ so as to make contact between the electrodes and the surface.

2. The catheter according to claim 1, wherein the strain-relief element comprises nitinol.

3. The catheter according to claim 1, wherein in response to being under a mechanical stress, the strain-relief element is configured to allow the spline to conform to the surface.

4. The catheter according to claim 1, wherein the strain-relief element has a shape selected from a list consisting of S-shape, U-Shape, Omega-shape, V-Shape, and Double V-shape.

5. The catheter according to claim 1, wherein the strain-relief element is positioned in a proximal-end of the at least one spline.

6. The catheter according to claim 1, wherein the strain-relief element is positioned in a distal-end of the at least one spline.

7. The catheter according to claim 1, wherein the at least one spline comprises:
at least one strain-relief element positioned at a proximal-end of the at least one spline; and
at least one strain-relief element positioned at a distal-end of the at least one spline.

8. The catheter according to claim 1, wherein a volume of the extended distal-end assembly is larger than a volume of the organ, and wherein the strain-relief element is configured to partially collapse so as to allow the splines to conform to the surface.

9. The catheter according to claim 1, wherein a volume of the extended distal-end assembly is smaller than a volume of the organ, and wherein the strain-relief element is configured to extend so as to allow the splines to conform to the surface.

10. A method for producing a catheter, comprising:
providing a shaft for insertion into an organ of a patient; and
coupling to the shaft an extendable distal-end assembly, which comprises multiple splines, wherein at least one spline comprises one or more electrodes and at least one strain-relief element.

11. The method according to claim 10, wherein coupling the distal-end assembly comprises forming at least one of the strain-relief element to have a shape selected from a list consisting of S-shape, U-Shape, Omega-shape, V-Shape, and Double V-shape.

12. The method according to claim 10, wherein coupling the distal-end assembly comprises positioning the strain-relief element in a proximal-end of the at least one spline.

13. The method according to claim 10, wherein coupling the distal-end assembly comprises positioning the strain-relief element in a distal-end of the at least one spline.

14. The method according to claim 10, wherein coupling the distal-end assembly comprises:
positioning at least one strain-relief element at a proximal end of the at least one spline; and
positioning at least one strain-relief element at a distal end of the at least one spline.
